# FASCICULE DE BREVET EUROPEEN

(11) **EP 3 295 965 B1**
(45) Date de publication et mention de la délivrance du brevet: **30.10.2019**
(21) Numéro de dépôt: 17190501.1
(22) Date de dépôt: 12.09.2017
(51) Int. Cl.: A61L 2/22, A61C 19/00

(54) **DISPOSITIF DE MODULE SUPPORT DE PORTE-INSTRUMENTS ROTATIFS**
VORRICHTUNG EINES HALTERUNGSMODULS FÜR SICH DREHENDE WERKZEUGHALTER
DEVICE OF A SUPPORT MODULE FOR ROTARY INSTRUMENT HOLDER

(30) Priorité: 14.09.2016 FR 1658597
(43) Date de publication de la demande: 21.03.2018
(73) Titulaire: Slupecki, Patrice, 45000 Orléans (FR); Slupecki, Annie, 45000 Orléans (FR)
(72) Inventeur: Slupecki, Patrice, 45000 Orléans (FR); Slupecki, Annie, 45000 Orléans (FR)
(74) Mandataire: Cabinet Bleger-Rhein-Poupon

(56) Documents cités:
- WO-A1-2011/128599
- US-A- 4 990 087
- US-A1- 2007 031 778
- US-B1- 6 217 329

## Description

La présente invention a pour objet un dispositif de module support de porte-instruments rotatifs, notamment, mais non limitativement des pièces à main, équipant, avec d'autres dispositifs de module analogues, un dispositif automatique de désinfection et d'entretien d'un porte-instruments rotatif.

La présente invention a également pour objet un dispositif automatique de désinfection et d'entretien d'un porte-instruments rotatif comportant ces dispositifs de module.

On connaît par le document WO 2011/128599, ainsi que par le document US 6 217 329, un dispositif automatique de désinfection de moteurs chirurgicaux, qui permet le nettoyage, la décontamination et le séchage, ainsi que la lubrification des éléments destinés à être mis en mouvement, et qui comporte, contenue dans une enceinte, une multiplicité de modules juxtaposés et solidarisés à au moins un moyen de réception disposé à l'intérieur de ladite enceinte, lesdits modules étant destinés chacun à recevoir un moteur chirurgical, et comprenant à cet effet des moyens de fixation dudit moteur chirurgical, ainsi que des moyens de connexion propres audit moteur chirurgical et aptes à permettre la diffusion desdits fluides, chacun des desdits modules comportant en outre des moyens réversibles de connexion, en vue de le relier à un ou deux modules voisins, et qui permettent d'alimenter lesdits modules en fluides, et de faire circuler lesdits fluides vers les autres modules connectés.

Le problème non résolu dans ce document concerne les modules qui renferment un moteur destiné à actionner le porte-instruments pendant l'opération de désinfection. En effet, pour que le résultat soit de qualité, il nécessaire que les pièces susceptibles d'être en mouvement dans la pièce à main, soient également mises en mouvement lors de l'opération de désinfection, et également de lubrification.

Or, cette opération de désinfection se déroule dans l'enceinte en milieu humide, peu compatible avec le fonctionnement électrique du moteur du module.

La présente invention a ainsi pour but de proposer un dispositif de module support de porte-instruments rotatif, permettant de résoudre ce problème, à savoir permettre l'entraînement en rotation d'un arbre disposé extérieurement au module support, au travers d'un moteur électrique disposé à l'intérieur dudit module, tandis que ledit module est exposé à un milieu humide.

L'une des solutions proposées par la présente invention consiste créer dans un module deux compartiments dans le prolongement l'un de l'autre, séparés par une paroi, dont l'un, fermé hermétiquement, contient le moteur, tandis que l'autre maintient un arbre mené dont une partie, destinée à être le support d'un porte-instruments rotatif, fait saillie extérieurement dudit module, l'arbre menant dudit moteur et ledit arbre mené étant liés en rotation par l'intermédiaire de moyens magnétiques disposés en regard de part et d'autre de ladite paroi qui est configurée pour ne pas engraver le champs magnétique créé.

Selon une autre solution proposée, le dispositif de module support de porte-instruments rotatifs selon l'invention, destiné à équiper avec d'autres modules analogues, un dispositif automatique de désinfection et d'entretien d'un porte-instruments rotatif, comprenant une enceinte, ladite enceinte enfermant une multiplicité de modules juxtaposés et solidarisés à au moins un moyen de réception disposé à l'intérieur de ladite enceinte, et où des modules comprennent d'une part des moyens de fixation de porte-instruments rotatifs ainsi que des moyens de connexion aptes à permettre la diffusion, dans le porte-instruments rotatif et entre les modules, de fluides de nettoyage et/ou de désinfection et/ou de lubrification ; et d'autre part un moteur apte à faire tourner le porte-instruments rotatif, et il se caractérise essentiellement en ce que ledit moteur est logé dans une cavité pratiquée dans ledit module, fermée par un couvercle traversé par l'arbre menant dudit moteur, ledit couvercle étant constitué de l'assemblage d'au moins deux pièces concentriques, une première de forme tubulaire destinée à être introduite dans l'ouverture de ladite cavité, et munie d'une part d'un système de joint périphérique apte à réaliser l'étanchéité avec ledit module, et d'autre part un système de joint interne apte à réaliser l'étanchéité avec ledit arbre, et une seconde pièce en forme d'anneau, introduite dans une feuillure périphérique pratiquée intérieurement dans ladite première pièce du côté de sortie dudit arbre, et comprenant, outre un système périphérique de joint apte à réaliser l'étanchéité avec ladite première pièce, un alésage central destiné à être traversé au plus juste par ledit arbre, à cet effet ce dernier présente des dimensions ajustées pour réaliser une étanchéité par serrage glissant.

L'étanchéité de la cavité, et donc la protection du moteur, est assurée au niveau du passage de l'arbre par une double barrière de joints.

Selon une caractéristique additionnelle du dispositif selon l'invention, le moteur est recouvert d'une gaine en matériau thermo rétractable.

Selon une autre caractéristique additionnelle du dispositif selon l'invention, l'intérieur de la cavité dans laquelle est placé le moteur, est rempli d'un matériau de comblement.

Selon une autre caractéristique additionnelle du dispositif selon l'invention, le matériau de comblement consiste en une résine semi-rigide ou un gel.

Les avantages et les caractéristiques du dispositif selon l'invention, ressortiront plus clairement de la description qui suit et qui se rapporte au dessin annexé, lequel en représente un mode de réalisation non limitatif.

Dans le dessin annexé:
- la figure 1 représente un dispositif automatique de désinfection de porte-instruments rotatifs, comprenant des dispositifs de modules selon l'invention.
- la figure 2 représente une vue schématique partielle en coupe et en éclaté d'un dispositif de module selon l'invention.
- la figure 3 représente une vue schématique partielle en coupe du même dispositif de module.
- la figure 4 représente une vue schématique et en coupe d'une autre solution au problème posé.

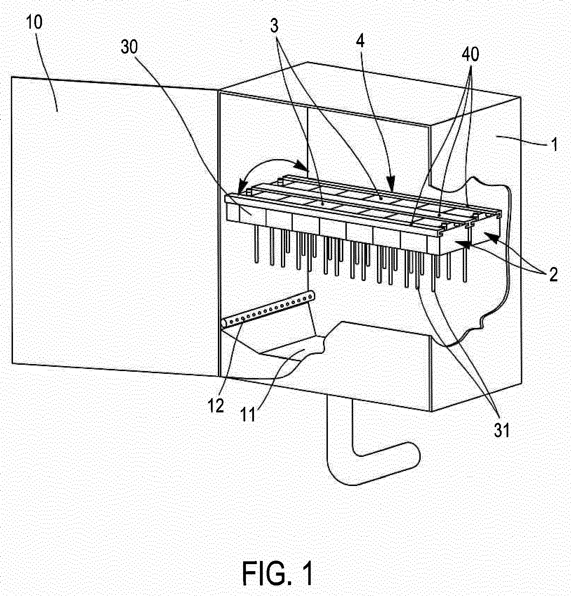

En référence à la figure 1, on peut ainsi voir un dispositif automatique de désinfection d'instruments chirurgicaux tel que celui décrit dans le document WO 2011/128599. Il comprend essentiellement, une enceinte 1 munie d'une porte 10, et renfermant des moyens 2 de maintien de porte-instruments rotatifs, non représentés, tels que des pièces à main, alimentés en fluides de nettoyage, de séchage et de lubrification, au travers de raccordements, également non représentés. Les moyens 2 de maintien consistent en des ensembles de modules 3 montés sur des moyens de réception 4 solidaires de l'enceinte 1, et consistant en des glissières 40.

Chaque module 3 comporte un bloc 30, en l'occurrence de forme cubique, duquel font saillie un nez de fixation d'une pièce à main, et des rampes 31 munies trous de pulvérisation.

Enfin, l'enceinte 1 comporte un fond 11 en forme de trémie pour permettre de la collecte des fluides après usage. De manière avantageuse, l'enceinte 1 est munie de rampes d'aspersion 12, dont une seule est visible sur la figure 1, destinées au nettoyage de l'enceinte 1 après les opérations de traitement.

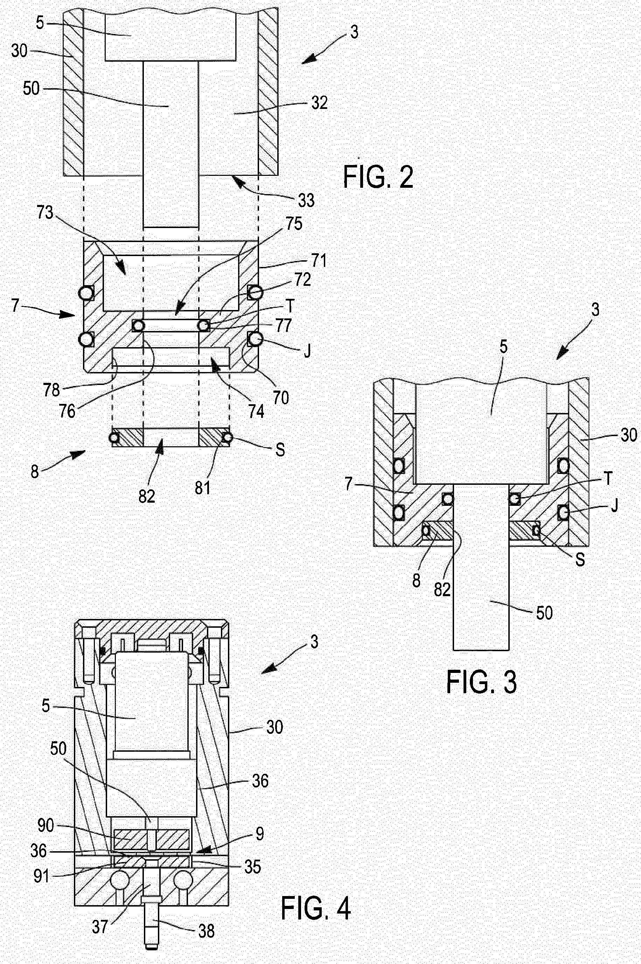

En référence maintenant aux figures 2 et 3, on peut voir un dispositif de module 3 selon l'invention, du type incorporant un moteur électrique 5 destiné à mettre en mouvement les pièces intérieures du porte-instruments rotatif porté par le module 3, au travers d'un arbre menant 50.

Sur ces figures 2 et 3, on peut voir que le bloc 30 comporte une cavité 32 dans laquelle est placé le moteur 5, et dont l'ouverture 33 est destinée à être fermée de manière étanche par un couvercle 6, conçu apte à permettre à l'arbre menant 50 de tourner.

Le couvercle 6 qui est destiné à être engagé dans l'ouverture 32, est composé de deux pièces 7 et 8 assemblables l'une à l'autre.

La pièce 7 se présente sous la forme générale d'un tube d'un diamètre extérieur lui permettant d'être introduite étroitement dans l'ouverture 33, l'étanchéité avec le bloc 30 étant réalisée au moyen d'un système de joint, comprenant en l'occurrence, non limitativement, deux joints toriques J, disposés chacun dans une gorge périphérique 70 pratiquée dans la paroi extérieure 71 de la pièce 7.

La pièce 7 comporte intérieurement une cloison 72 qui délimite d'un côté un logement 73 destiné à coiffer la partie extrême 51 du moteur 5 de laquelle fait saillie l'arbre menant 50, et de l'autre une cavité 74 destinée à loger la pièce 8.

La cloison 72 est percée centralement d'un alésage 75 pour le passage étroit de l'arbre 50, dans la paroi intérieure 76 duquel est pratiquée une gorge périphérique 77 garnie d'un joint torique T.

La pièce 8 se présente sous une forme annulaire, elle est d'épaisseur correspondant à la profondeur de la cavité 74, elle comporte d'une part une paroi extérieure 80 dans laquelle est creusée une cavité périphérique 81 logeant un joint S, permettant de réaliser l'étanchéité avec la paroi périphérique interne 78 de la cavité 74, et d'autre part un alésage central 82 dont le diamètre est ajusté à celui de l'arbre 50 en sorte de pouvoir réaliser une étanchéité par serrage glissant.

Les assemblages de la pièce 8 à la pièce 7, et de la pièce 7 au bloc 30, au travers des emboîtements au travers des différents joints J, T et S, permet de garantir une parfaite coaxialité de l'arbre 50 et de l'alésage 82, sachant que par ailleurs, la position du moteur 5 est maintenue et stabilisée dans le bloc 30.

En référence à la figure 9, on peut voir une autre solution au problème de l'étanchéité, utilisant le magnétisme.

On peut voir un module 3, comprenant un bloc 30 dans lequel sont pratiqués deux compartiments 34 et 35 dans le prolongement l'un de l'autre, et séparés par une paroi 36. Le compartiment 34 est entièrement hermétique et il loge le moteur 5, tandis que le compartiment 35 comporte un palier 37 supportant un arbre mené 38 qui fait saillie du bloc 30, et dont la partie extérieure à ce bloc 30 est destinée à recevoir un porte-instruments rotatif.

On notera que le compartiment 35 peut être créé dans le bloc 30, ou bien venir de l'adjonction au bloc 30 d'un élément rapporté.

L'arbre menant 50 du moteur 5 et l'arbre mené 38, sont liés par l'intermédiaire d'une liaison magnétique 9, laquelle comprend un volant 90 solidaire de l'arbre menant 50 et un plateau 91 duquel est solidaire l'arbre mené 38, le volant 90 et le plateau 91 étant en regard l'un de l'autre, de part et d'autre de la paroi 36, et munis de moyens magnétiques tels que des aimants, non représentés.

La paroi 36 est bien entendu configurée pour ne pas entraver le champs magnétique créé, et permettre l'entraînement du plateau 91 et donc de l'arbre mené 38, elle est notamment de faible épaisseur.

## Revendications

1. Dispositif de module (3) support de porte-instruments rotatifs, destiné à équiper avec d'autres modules analogues (3), un dispositif automatique de désinfection et d'entretien d'un porte-instruments rotatif, comprenant une enceinte (1), ladite enceinte (1) enfermant une multiplicité de modules (3) juxtaposés et solidarisés à au moins un moyen de réception (4) disposé à l'intérieur de ladite enceinte (1), et où des modules (3) comprennent d'une part des moyens de fixation de porte-instruments rotatifs ainsi que des moyens de connexion aptes à permettre la diffusion, dans le porte-instruments rotatif et entre les modules (3), de fluides de nettoyage et/ou de désinfection et/ou de lubrification ; et d'autre part un moteur (5) apte à faire tourner le porte-instruments rotatif, ledit moteur (5) est logé dans une cavité (32) pratiquée dans ledit module (3), fermée par un couvercle (6) traversé par l'arbre menant (50) dudit moteur (5), ledit couvercle (6) étant constitué de l'assemblage d'au moins deux pièces concentriques (7, 8), **caractérisé en ce qu'**une première (7) de forme tubulaire destinée à être introduite dans l'ouverture (33) de ladite cavité (32), et munie d'une part d'un système de joint périphérique (J) apte à réaliser l'étanchéité avec ledit module (3), et d'autre part un système de joint interne (T) apte à réaliser l'étanchéité avec ledit arbre (50), et une seconde pièce (8) en forme d'anneau, introduite dans une feuillure (74) périphérique pratiquée intérieurement dans ladite première pièce (7) du côté de sortie dudit arbre (50), et comprenant, outre un système périphérique de joint (S) apte à réaliser l'étanchéité avec ladite première pièce (7), un alésage central (75) destiné à être traversé au plus juste par ledit arbre (50), à cet effet ce dernier présente des dimensions ajustées pour réaliser une étanchéité par serrage glissant.

2. Dispositif de module (3) selon la revendication 1, **caractérisé en ce que** le moteur (5) est recouvert d'une gaine en matériau thermo rétractable.

3. Dispositif de module selon la revendication 1 ou la revendication 2, **caractérisé en ce que** l'intérieur de la cavité (32) dans laquelle est placé le moteur (5), est rempli d'un matériau de comblement.

4. Dispositif de module selon la revendication 3, **caractérisé en ce que** le matériau de comblement consiste en une résine semi-rigide ou un gel.

5. Dispositif automatique de désinfection et d'entretien d'un porte-instruments rotatif, **caractérisé en ce qu'**il est équipé de modules (3) selon l'une quelconque des revendications 1 à 4.

## Patentansprüche

1. Modulvorrichtung (3) zum Tragen von drehbaren Instrumentenhaltern, dazu bestimmt, mit anderen ähnlichen Modulen (3) eine automatische Vorrichtung zur Desinfektion und Wartung eines drehbaren Instrumentenhalters auszustatten, umfassend eine Kammer (1), wobei die besagte Kammer (1) eine Vielzahl von Modulen (3) einschließt, die nebeneinander angeordnet und mit mindestens einem in der besagten Kammer (1) angeordneten Aufnahmemittel (4) fest befestigt sind, und wobei die Module (3) einerseits Mittel zum Befestigen von drehbaren Instrumentenhaltern sowie Verbindungsmittel umfassen, die geeignet sind, die Verbreitung im drehbaren Instrumentenhalter und zwischen den Modulen (3) von Reinigungs- und/oder Desinfektions- und/oder Schmiermitteln zu erlauben; und andererseits einen Motor (5), der geeignet ist, den drehbaren Instrumentenhalter drehen zu lassen, wobei der besagte Motor (5) in einem Hohlraum (32) untergebracht ist, der in dem besagten Modul (3) vorgesehen, durch einen Deckel (6), der von der Antriebswelle (50) des besagten Motors (5) durchsetzt wird, verschlossen ist, wobei der besagte Deckel (6) aus der Zusammenfügung von mindestens zwei konzentrischen Teilen (7, 8) besteht, **dadurch gekennzeichnet, dass** ein erstes rohrförmiges Teil (7) dazu bestimmt ist, in die Öffnung (33) des besagten Hohlraums (32) eingeführt zu werden, und einerseits mit einem umlaufenden Dichtungssystem (J), das zum Sichern der Abdichtung mit dem besagten Modul (3) geeignet ist, und andererseits ein inneres Dichtungssystem (T), das geeignet ist, die Abdichtung mit der besagten Welle (50) zu sichern, versehen ist, und ein zweites ringförmiges Teil (8) in einem umlaufenden Falz (74), der innen in dem besagten ersten Teil (7) an der Ausgangsseite der besagten Welle (50) vorgesehen ist, eingeführt ist und neben einem umlaufenden Dichtungssystem (S), das geeignet ist, die Abdichtung mit dem besagten ersten Teil (7) zu sichern, eine zentrale Bohrung (75) umfasst, die dazu bestimmt ist, passgenau von der besagten Welle (50) durchsetzt zu werden, wobei letztere zu diesem Zweck angepasste Abmessungen aufweist, um eine Abdichtung durch Gleitspannung zu sichern.

2. Modulvorrichtung (3) nach Anspruch 1, **dadurch gekennzeichnet, dass** der Motor (5) mit einer Hülle aus wärmeschrumpfbarem Material bedeckt ist.

3. Modulvorrichtung nach Anspruch 1 oder 2, **dadurch gekennzeichnet, dass** der Innenraum des Hohlraums (32), in dem der Motor (5) angebracht ist, mit einem Füllmaterial gefüllt ist.

4. Modulvorrichtung nach Anspruch 3, **dadurch gekennzeichnet, dass** das Füllmaterial aus einem halbstarren Harz oder einem Gel besteht.

5. Automatische Vorrichtung zur Desinfektion und Wartung eines drehbaren Instrumentenhalters, **dadurch gekennzeichnet, dass** sie mit Modulen (3) nach irgendeinem der Ansprüche 1 bis 4 ausgestattet ist.

## Claims

1. Module device (3) for supporting a rotary instrument-holder, intended to be provided with other analogous modules (3) on an automatic device for disinfecting and maintaining a rotary instrument-holder, comprising an enclosure (1), said enclosure (1) enclosing a plurality of modules (3) juxtaposed to and made integral with at least one receiving means (4) arranged inside said enclosure (1), and wherein the modules (3) comprise, on the one hand, means for fixing rotary instrument-holders as well as connecting means capable of permitting the diffusion, in the rotary instrument-holder and between the modules (3), of cleaning and/or disinfecting and/or lubricating fluids; and, on the other hand, a motor (5) capable of causing the rotary instrument-holder to rotate, said motor (5) being accommodated in a cavity (32) formed in said module (3), closed by a cover (6) through which passes the driving shaft (50) of said motor (5), said cover (6) consisting of the assembly of at least two concentric parts (7, 8), wherein a first part (7) having a tubular shape is intended to be inserted into the opening (33) of said cavity (32) and provided, on the one hand, with a peripheral seal system (J) capable of ensuring the tightness with said module (3) and, on the other hand, an internal seal system (T) capable of ensuring the tightness with said shaft (50), and a ring-shaped second part (8) inserted into a peripheral rabbet (74) is formed internally in said first part (7) on the outlet side of said shaft (50) and comprises, in addition to a peripheral seal system (S) capable of ensuring the tightness with said first part (7), a central bore (75) intended to be crossed as tightly as possible by said shaft (50), to this end the latter has dimensions adjusted to ensure the tightness by slidable clamping.

2. Module device (3) according to claim 1, wherein the motor (5) is covered with a sheath made of heat-shrinkable material.

3. Module device according to claim 1 or claim 2, wherein the interior of the cavity (32), in which the motor (5) is placed, is filled with a filler material.

4. Module device according to claim 3, wherein the filler material consists of a semi-rigid resin or a gel.

5. Automatic device for disinfecting and maintaining a rotary instrument-holder, wherein it is provided with modules (3) according to any one of claims 1 to 4.
